**Europäisches Patentamt**

**European Patent Office**

**Office européen des brevets**

(19)

(11) **EP 1 388 800 A1**

(12) **EUROPÄISCHE PATENTANMELDUNG**

(43) Veröffentlichungstag:
**11.02.2004 Patentblatt 2004/07**

(51) Int Cl.[7]: **G06F 19/00**

(21) Anmeldenummer: **02017469.4**

(22) Anmeldetag: **05.08.2002**

| | |
|---|---|
| (84) Benannte Vertragsstaaten:<br>**AT BE BG CH CY CZ DE DK EE ES FI FR GB GR IE IT LI LU MC NL PT SE SK TR**<br>Benannte Erstreckungsstaaten:<br>**AL LT LV MK RO SI**<br><br>(71) Anmelder: **Universität zu Lübeck**<br>**23538 Lübeck (DE)**<br><br>(72) Erfinder:<br>• **Martinetz, Thomas, c/o Inst.für Neuro-u. Bioinf.**<br>**23569, Lübeck (DE)** | • **Gewehr, Jan E., c/o Inst. für Neuro-u.Bioinform.**<br>**23569, Lübeck (DE)**<br>• **Kim, Jan T. c/o Inst. für Neuro-u. Bioinform.**<br>**23569, Lübeck (DE)**<br><br>(74) Vertreter: **Biehl, Christian, Dipl.-Phys. et al**<br>**Boehmert & Boehmert,**<br>**Anwaltssozietät,**<br>**Niemannsweg 133**<br>**24105 Kiel (DE)** |

(54) **Verfahren zum Bestimmen potentieller sequenzspezifischer Protein-Bindungsstellen**

(57) Verfahren zum Bestimmen potentieller sequenzspezifischerProtein-ProteinundProtein-DNA-Bindungsstellen eines Objekts an eine Sequenz von Modulen mit Hilfe einer in einem Computersystem implementierten Matrix m zur Vorhersage, ob eine vorbestimmte Protein-Sequenz als Teilwort aus Modulen $b \in A$ mit der Länge L für ein gegebenes Objekt, ein Protein, eine ausreichende Bindungsenergie besitzt, wobei aufgrund experimentell bestimmter Bindungsstellen $w_B^E$ die Matrix m sowie ein Minimumwert $S_{min}$ bestimmt werden, anhand derer für ein Sequenzwort $w^i$ an der i-ten Position der Sequenz ein Score-Wert

$$S(w^i) = m\, w^i = \Sigma_l\, \Sigma_b\, m_{bl} w_{bl}^i$$

berechnet wird, aufgrund dessen das Wort $w^i$ als Bindungswort und die Position i als Bindungsstelle bezeichnet werden, falls $S(w^i) \geq S_{min}$ gilt, und die Menge $\mathbf{B} \subseteq \mathbf{W}$ der Bindungsworte durch diejenigen Worte auf einer Kugelkappe einer $(|\mathbf{A}| -1) \cdot L$-dimensionalen Hyperkugel gegeben ist, die im Halbraum $\mathbf{p}\,\mathbf{w} \geq E_B$ liegen, und durch die Bestimmung der m, durch eine Binding-Matrix $\mathbf{q}$, einer $|\mathbf{A}| \times L$-Matrix, und des Minimumswerts $S_{min}$ durch einen Schwellwert $q_0 \in \mathbb{R}$, welche durch eine Optimierungsschätzung der durch $\mathbf{p} \cdot \mathbf{w} = E_B$ gegebenen Ebene durch Optimierung in den Variablen $\mathbf{q}$ und $q_0$ dadurch bestimmt werden, daß der durch $\mathbf{q} \cdot (\mathbf{w} - \bar{w}) \geq q_0$ gegebene Halbraum eine Kugelkappe von der durch alle Worte $\mathbf{w}$ gegebenen Hyperkugel abtrennt, auf der alle experimentell verifizierten Bindungsworte liegen und deren Oberfläche als Maß für die Gesamtzahl der Worte auf der Kappe minimiert wird.

*Fig. 3*

EP 1 388 800 A1

## Beschreibung

Die Erfindung betrifft ein Verfahren zum Bestimmen potentieller sequenzspezifischer Protein-Bindungsstellen nach dem Oberbegriff des Hauptanspruches.

Die Vorhersage insbesondere der Bindungsstellen von DNA-Bindungsproteinen ist ein in der Bioinformatik wichtiger Aspekt. Zum Beispiel sind die Bindungsstellen von Transkriptionsfaktoren die Schlüsselelemente von regulativen Netzwerken und bestimmen die Anordnung von Genen auf einem Genom. Normalerweise sind für ein gegebenes DNA-Bindungsprotein nur einige Bindungssequenzen experimentell bekannt. Aus dem Artikel "Finding protein-binding sites in DNA sequences: the next generation", TIBS 22, März 1997, Komelie Frech at al., ist bereits bekannt, Gewichtungsmatrizen einzusetzen, die "scores" als quantitative Bewertung der Wahrscheinlichkeit einer Protein-DNA-Bindung für jede Position liefern. Eine Anzahl von kommerziell erhältlichen Computerprogrammen wird angegeben.

Aufgabe der vorliegenden Erfindung ist nun eine lineare Klassifikationsmatrix zu schaffen, mit der die Bindungsstellen und ihre Positionen ermittelt werden. Dies wird durch eine Binding-Matrix nach dem geltenden Hauptanspruch erreicht. Die Unteransprüche geben vorteilhafte Ausführungsformen der Erfindung wieder.

Für den Fall, dass eine lineare Sequenz von Modulen vorgegeben ist, zum Beispiel eine Sequenz molekularer Bausteine wie eine DNA oder ein Protein, kann eine Buchstabensequenz der Länge N aus einzelnen molekularen Bausteinen als die zu untersuchende Sequenz vorgegeben werden. Vorhanden ist außerdem ein Objekt, welches an die lineare Sequenz binden kann. Im typischen Anwendungsfall handelt es sich bei der Sequenz um eine DNA-Sequenz und bei dem Objekt um einen Transkriptionsfaktor. In der folgenden Darstellung wird zur Vereinfachung auf diesen Anwendungsfall als Beispiel zurückgegriffen.

Die Bindungsstärke bzw. Bindungsenergie an dem Ort der Bindung hängt allein von der Subsequenz ab, die das Objekt an dem Ort vorfindet. Die Länge der Subsequenz, des Objekt "sieht" ist mit L bezeichnet. Ein Transkriptionsfaktor soll nun auf sein Bindungsverhalten - typischerweise an einer Subsequenz von 10 bis 20 Nukleinsäuren - untersucht werden. Diese Subsequenz wird als Wort bezeichnet und das Wort $w_i$ am Ort i ist gegeben durch die Buchstaben (Module) $b_i$ , $b_{i+1}$ bis $b_{i+L-1}$. Orte auf der Sequenz, auf denen die Bindungsenergie des Objektes einen gewissen Schwellwert überschreitet, heißen Bindungsstellen (binding sites).

Ein DNA-bindendes Protein wie ein Transkripitonsfaktor hat nun an solchen Bindungsstellen eine ausreichend starke Bindung, und kann dort eine Funktion erfüllen.

Im folgenden wird die mathematische Herleitung der Richtigkeit des gewählten Ansatzes anhand einer Zeichnung erfolgen. Dabei zeigt:

Fig. 1     ein Wort als Objekt auf einer Sequenz von Modulen,

Fig. 2     eine schematische Kugelkappe auf einer Hyperkugel, und

Fig. 3     eine Veranschaulichung einer Dicke der Kugelkappe.

Gegeben sei eine lineare Sequenz $b_i$, i=1, ..., N von Modulen $b_i \in$ **A**, z.B. eine Sequenz molekularer Bausteine wie eine DNA (Module sind also Nukleinsäuren, $b_i \in \{A,C,G,T\}$) oder ein Protein (Module sind Aminosäuren). **A** kann als Alphabet und $b_i$, i=1, ..., N als Buchstabensequenz der Länge N betrachtet werden.

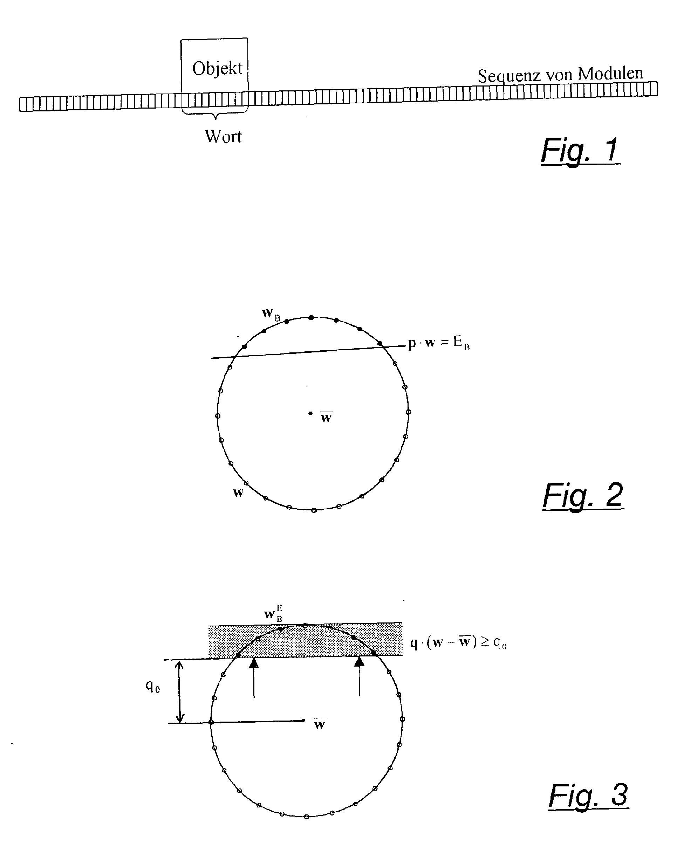

Gegeben ist außerdem ein Objekt, welches an die lineare Sequenz binden kann, z.B. ein Protein wie ein Transkriptionsfaktor, der an eine DNA-Sequenz bindet, oder auch ein Protein, welches an ein anderes Protein bindet. Die Bindungsstärke bzw. Bindungsenergie am Ort i, i=1,...N der Sequenz hängt allein von der Subsequenz ab, die das Objekt um den Ort i vorfindet. Die Länge der Subsequenz, die das Objekt "sieht", bezeichnen wir mit L. Ein Transkriptionsfaktor "sieht" typischerweise eine Subsequenz von 10-20 Nukleinsäuren. Diese Subsequenzen nennen wir Worte, und das Wort $w_i$ am Ort i ist gegeben durch die Buchstaben (Module) $b_i$, $b_{i+1}$, ... $b_{i+L-1}$. In Fig. 1 ist eine solche Subsequenz beispielhaft dargestellt.

Orte auf der Sequenz, auf denen die Bindungsenergie des Objekts einen gewissen Schwellwert überschreitet, heißen "binding sites". Zum Beispiel hat ein DNA-bindendes Protein wie ein Transkriptionsfaktor an solchen Orten eine ausreichend starke Bindung, um dort eine Funktion zu erfüllen.

Das zu lösende Problem ist die Prädiktion solcher "binding sites", zum Beispiel auf einem Genom. Dazu ist es notwendig, diejenigen Worte $\mathbf{w}_B$ aus der Menge **W** der K =$|\mathbf{A}|^L$ möglichen Worte zu bestimmen, an denen das Objekt mit ausreichender Bindungsenergie bindet. Zur Bestimmung dieser Menge **B** von k =$|\mathbf{B}|$ "binding words" $\mathbf{w}_B$ steht allerdings in der Regel nur eine um Größenordnungen kleinere Menge $B_E \subseteq B$ experimentell verifizierter "binding words" $\mathbf{w}_B^E$ zur Verfügung. Allein aus diesen $\mathbf{w}_B^E$ muß auf das Bindungsverhalten des Objektes geschlossen werden.

Jedes Wort $\mathbf{w} \in \mathbf{W}$ läßt sich durch einen Vektor der Länge $|\mathbf{A}| \cdot L$ beschreiben, mit $|\mathbf{A}|$ Einträgen für jeden der L Buchstaben des Wortes **w**. Von den $|\mathbf{A}|$ Einträgen für jeden Buchstaben ist einer "Eins" und alle anderen "Null", entsprechend dem dortigen Buchstaben. Zum Beispiel ist das Wort "ACT" auf der DNA mit Buchstaben aus $b \in \{A,C,G,T\}$ gebildet und wird entsprechend durch (1000,0100,0001) kodiert.

Experimentell ist gut verifiziert, daß bei der DNA-Bindung eines Proteins, zum Beispiel eines Transkriptionsfaktors, jede Base $b_i \in \{A, C, G, T\}$ in guter Näherung unabhängig von ihren Nachbarbasen seinen Beitrag $p_i$ zur Bindungsenergie E liefert. Dann ist das Bindungsverhalten des Proteins an die DNA durch den Vektor $\mathbf{p} = (p_{A1}, p_{C1}, p_{G1}, p_{T1}, p_{A2}, ..., p_{TL})$ vollständig beschrieben, und die Bindungsenergie bei der Bindung an ein Wort $\mathbf{w} = (w_{A1}, w_{C1}, w_{G1}, w_{T1}, w_{A2}, ..., w_{TL})$, mit $w_{bl} \in \{0,1\}, b \in \mathbf{A}, 1 = 1, ..., L$ entsprechend der obigen Kodierung, ist gegeben durch

$$E(\mathbf{w}) = \sum_{l=1}^{L} \sum_{b \in A} p_{bl} w_{bl} = \mathbf{p} \cdot \mathbf{w}$$

Falls diese Bindungsenergie einen Mindestwert $E_B$ überschreitet, so ist w ein "binding word" und es gilt $\mathbf{w} \in \mathbf{B}$. Die Menge $\mathbf{B} \subseteq \mathbf{W}$ der "binding words" ist also bestimmt durch den Vektor $\mathbf{p}$ und den Schwellwert $E_B$.

Im $|\mathbf{A}| \cdot L$ -dimensionalen Raum der als Vektoren kodierten Worte liegen alle Worte $\mathbf{w} \in \mathbf{W}$ auf einer Hyperkugel um den Ursprung mit Radius $|\mathbf{w}|^2 = L$. Der Schwerpunkt aller Worte ist gegeben durch $\overline{\mathbf{w}}$ mit $\overline{w}_{bl} = 1/|\mathbf{A}|$ für alle $b \in \mathbf{A}, 1 = 1, ..., L$. Mit $(\mathbf{w} - \mathbf{w'}) \cdot \overline{\mathbf{w}} = 0$ für alle $\mathbf{w}, \mathbf{w'} \in \mathbf{W}$ liegen alle Worte auf einer Hyperebene senkrecht zu $\overline{\mathbf{w}}$. Des weiteren liegen alle Worte auf einer $(|\mathbf{A}| - 1) \cdot L$ - dimensionalen Hyperkugel um den Schwerpunkt $\overline{\mathbf{w}}$ mit Radius $|\mathbf{w} - \overline{\mathbf{w}}|^2 = L(1 - 1/|\mathbf{A}|)$. Auf dieser $(|\mathbf{A}| - 1) \cdot L$ -dimensionalen Hyperkugel sind die Worte aus Symmetriegründen gleichförmig verteilt. Der Vektor p zusammen mit dem Schwellwert $E_B$ beschreibt mit $\mathbf{p} \cdot \mathbf{w} = E_B$ eine Hyperebene, die den $|\mathbf{A}| \cdot L$ - dimensionalen Raum der Worte in zwei Halbräume teilt. Die Menge $\mathbf{B} \subseteq \mathbf{W}$ der "binding words" ist dann durch diejenigen Worte auf der $(|\mathbf{A}| - 1) \cdot L$ -dimensionalen Hyperkugel gegeben, die im Halbraum $\mathbf{p} \cdot \mathbf{w} \geq E_B$ liegen. In der Fig. 2 soll dies verdeutlicht werden.

Zur Prädiktion potentieller "binding sites" auf der gegebenen Sequenz von Modulen, z.B. von "transcription factor binding sites" auf einem Genom, muß die durch $\mathbf{p}$ gegebene Teilung der $(|\mathbf{A}| - 1) \cdot L$ -dimensionalen Hyperkugel in eine "Kugelkappe" mit "binding words" und eine mit "non-binding words" bestimmt werden.

Dazu stehen in der Regel nur einige wenige experimentell verifizierte "binding words" $\mathbf{w}_B^E \in \mathbf{B}_E \subseteq \mathbf{B}$ zur Verfügung.

Das vorgeschlagene neue Verfahren, die *Binding-Matrix,* beruht auf dem systematischen Ansatz, aus den experimentell verifizierten binding words die Maximum Likelihood Schätzung für die durch p und $E_B$ gegebene Ebne abzuleiten. Unter der Annahme, daß alle binding words $w_B$ mit $\mathbf{p} \cdot \mathbf{w} \geq E_B$ die biologische Funktion (spezifische Bindung des Objekts/Proteins) gleich gut erfüllen können, ist die gesuchte Schätzung durch diejenige Ebene gegeben, bei der alle experimentell verifizierten binding words auf derselben Seite der Ebene liegen und gleichzeitig ein möglichst kleiner Teil der Hyperkugel aller Worte auf der Seite der Ebne liegen, auf der die binding words liegen. Anders gesagt trennt die gesuchte Ebne eine möglichst kleine Kappe von der Hyperkugel ab, so daß alle binding words auf der Kugelkappe liegen. Dies ist in Fig. 3 skizziert. Die Oberfläche dieser Kugelkappe kann als Maßzahl für die Gesamtzahl der Worte auf dieser Kappe dienen.

Für den Fall, dass alle "binding words" bekannt sind und damit $\mathbf{B}_E = \mathbf{B}$ gilt, liefert dieses Verfahren bis auf Abweichungen aufgrund der diskreten Verteilung der Worte die durch $\mathbf{p}$ und $E_B$ definierte "Kugelkappe". Die Menge der Worte auf der durch diese Hyperebene abgetrennten "Kugelkappe" ist dann gleich $\mathbf{B}$. Damit ist dieses Klassifikationsverfahren selbst konsistent.

Die *Binding-Matrix* $\mathbf{q}$ ist eine $|\mathbf{A}| x L$-Matrix bzw. ein $|\mathbf{A}| \cdot L$-dimensionaler Vektor, der wie $\mathbf{p}$ die Orientierung einer Hyperebene im $|\mathbf{A}| \cdot L$ -dimensionalen Einbettungsraum $R^{|A| \cdot L}$ definiert. Da $\mathbf{q}$ nur die Orientierung bestimmen soll, ist $\mathbf{q}$ zu $\mathbf{q}^2 = 1$ normiert. Die Hyperebene ist dann durch die Punkte $\mathbf{x} \in R^{|A| \cdot L}$ mit $\mathbf{q} \cdot (\mathbf{x} - \overline{\mathbf{w}}) = q_0$ gegeben, wobei $q_0 \in R$ den Abstand zum Schwerpunkt $\overline{\mathbf{w}}$ bestimmt. Um die Oberfläche der durch $\mathbf{q} \cdot (\mathbf{w} - \overline{\mathbf{w}}) \geq q_0$ von der Hyperkugel aller Worte abgetrennten Kappe zu minimieren, und somit die sngestrebte Maximum Likelihood Schätzung zu relisieren, muss q0 maximiert werden (vgl. Fig. 3). Wir suchen also dasjenige Paar $\mathbf{q}, q_0$, für das $\mathbf{q} \cdot (\mathbf{w}_B^E - \overline{\mathbf{w}}) \geq q_0$ gilt für alle $\mathbf{w}_B^E$ und für welches $q_0$ maximal wird. Je größer $q_0$, desto kleiner die Kugelkappe.

Die *Binding-Matrix* $\mathbf{q}$ sowie $q_0$ werden durch "Konvexe Optimierung" in den Variablen $\mathbf{q}, q_0$ bestimmt. Die Zielfunktion ist mit $q_0$ linear. Die Nebenbedingungen $\mathbf{q} \cdot (\mathbf{w}_B^E - \overline{\mathbf{w}}) \geq q_0$ für alle $\mathbf{w}_B^E \in \mathbf{B}_E$ sind ebenfalls linear. Allein die Nebenbedingung $\mathbf{q}^2 = 1$ ist nicht linear, aber konvex. Zur Lösung dieses linearen Optimierungsproblems mit konvexen Nebenbedingungen gibt es gängige numerische Lösungsverfahren.

Ist $\mathbf{q}, q_0$ über konvexe Optimierung bestimmt, so gelten alle Worte $\mathbf{w}$, für die $\mathbf{q} \mathbf{w} \geq \theta$ mit $\theta = q_0 + \mathbf{q} \overline{\mathbf{w}}$ gilt, als "binding words". Aufgrund der Nebenbedingungen $\mathbf{q} \cdot (\mathbf{w}_B^E - \overline{\mathbf{w}}) \geq q_0$ gehören auf jeden Fall alle experimentell verifizierten "binding words" dazu. Durch Verringerung des Schwellwertes $\theta$ kann die Zahl $k_q(\theta)$ der als "binding words" klassifizierten Worte erhöht werden. Dadurch steigt die Sensitivität, bis bei $\theta = \theta_{all}$ alle $k = |\mathbf{B}|$ "binding words" als solche erkannt werden und die Sensitivität ihren Maximalwert erreicht hat. Dies geht natürlich auf Kosten der Selektivität. Die Zahl der falsch klassifizierten Worte ("false positivs") liegt dann bei $k_q(\theta_{all}) - k$. Die Selektivität liegt in diesem "Arbeitspunkt" dann bei $1 - (k_q(\theta_{all}) - k) / K$ mit K als der Gesamtzahl der Worte.

Ist die *Binding-Matrix* $\mathbf{q}$ mit den vorhandenen experimentell verifizierten "binding words" $\mathbf{w}_B^E$ be-

stimmt, so können auf der gegebenen linearen Sequenz (Genom, Protein,...) von Modulen $b_i$, i=1, ..., N (Nukleinsäuren, Aminosäuren,...) die potentiellen "binding sites" bestimmt werden. Dazu wird auf jeder Position der Sequenz der zu dem dort vorliegenden Wort $w_i = (b_i, b_{i+1}, ...b_{i+L-1})$ der "score" $q \cdot w_i = s_i$ bestimmt. Überschreitet der "score" den vorgegebenen Schwellwert $\theta$, so wird $w_i$ als "binding word" und Position i somit als "binding site" klassifiziert. Diese Vorgehensweise entspricht dem derzeit am häufigsten verwendeten Verfahren zur Bestimmung von "transcription factor binding sites", dem sogenannten Profilmatrix-Ansatz. Die Profilmatrix ist wie die *Binding-Matrix* eine 4xL-Matrix.

Im Gegensatz zur *Binding-Matrix,* deren Elemente durch die oben beschriebene Optimierungsprozedur bestimmt wurde, stehen bei der Profilmatrix in jeder Spalte i=1,...L lediglich die relativen Häufigkeiten des Auftretens der Basen b in den experimentell bestimmten "binding words". Die Profilmatrix in entsprechender Vektorschreibweise ist damit gegeben durch $\overline{w}_B^E$. Auch hier wird für jedes Wort $w_i$ ein "score" $\overline{w}_B^E \cdot w_i$ berechnet, und falls dieser einen Mindestwert überschreitet, so gilt das Wort $w_i$ als "binding word" und die Position i als "binding site".

**Patentansprüche**

1. Verfahren zum Bestimmen potentieller sequenzspezifischer Protein-Protein und Protein-DNA-Bindungsstellen eines Objekts an eine Sequenz von Modulen mit Hilfe einer in einem Computersystem implementierten Matrix m zur Vorhersage, ob eine vorbestimmte Protein-Sequenz als Teilwort aus Modulen b $\in$ A mit der Länge L für ein gegebenes Objekt, ein Protein, eine ausreichende Bindungsenergie besitzt,
wobei aufgrund experimentell bestimmter Bindungsstellen $w_B^E$ die Matrix m sowie ein Minimumwert $S_{min}$ bestimmt werden, anhand derer für ein Sequenzwort w' an der i-ten Position der Sequenz ein Score-Wert

$$S(w^i) = m \, w^i = \sum_1 \sum_b m_{bl} w^i_{bl}$$

berechnet wird, aufgrund dessen das Wort $w^i$ als Bindungswort und die Position i als Bindungsstelle bezeichnet werden, falls $S(w^i) \geq S_{min}$ gilt
**dadurch gekennzeichnet, daß**
die Menge **B** $\subseteq$ **W** der Bindungsworte durch diejenigen Worte auf einer Kugelkappe einer (| **A** |-1)$\cdot$ L -dimensionalen Hyperkugel gegeben ist, die im Halbraum $\mathbf{p} \cdot \mathbf{w} \geq E_B$ liegen, und
durch die Bestimmung der m, durch eine Binding-Matrix **q**, einer |**A**| x L-Matrix, und des Minimumswerts $S_{min}$ durch einen Schwellwert $q_0 \in \mathbb{R}$, welche durch eine Optimierungsschätzung der durch $\mathbf{p} \cdot \mathbf{w} = E_B$ gegebenen Ebene durch Optimierung in den Variablen **q** und $q_0$ dadurch bestimmt werden, daß der durch $\mathbf{q} \cdot (\mathbf{w} - \overline{w}) \geq q_0$ gegebene Halbraum eine Kugelkappe von der durch alle Worte **w** gegebenen Hyperkugel abtrennt, auf der alle experimentell verifizierten Bindungsworte liegen und deren Oberfläche als Maß für die Gesamtzahl der Worte auf der Kappe minimiert wird.

2. Verfahren nach Anspruch 1, **dadurch gekennzeichnet, daß** anstelle der Bedingung $\mathbf{q} \cdot (\mathbf{w} - \overline{w}) \geq q_0$ die modifizierte Bedingung $\mathbf{q} \cdot \mathbf{w} \geq \Theta$ eingesetzt wird, so daß durch von $\Theta = q_0 + \mathbf{q} \cdot \overline{w}$ abweichende Einstellungen des Parameters $\Theta$ eine größere Sensitivität bei gleichzeitig verringerter Selektivität oder aber eine größere Selektivität bei gleichzeitig verringerter Sensitivität erreicht werden können.

3. Verfahren nach Anspruch 1 oder 2, **dadurch gekennzeichnet, daß** die Optimierung in den Variablen **q**, $q_0$ durch konvexe Optimierung erfolgt.

4. Verfahren nach einem der vorangehenden Ansprüche, **dadurch gekennzeichnet, daß** die Bindungsstellen Bindungsstellen von Transkriptionsfaktoren auf DNA-Sequenzen sind.

Objekt

Sequenz von Modulen

Wort

*Fig. 1*

$\mathbf{w}_B$

$\mathbf{p} \cdot \mathbf{w} = E_B$

$\overline{\mathbf{w}}$

$\mathbf{w}$

*Fig. 2*

$\mathbf{w}_B^E$

$\mathbf{q} \cdot (\mathbf{w} - \overline{\mathbf{w}}) \geq q_0$

$q_0$

$\overline{\mathbf{w}}$

*Fig. 3*

**Europäisches Patentamt**

# EUROPÄISCHER RECHERCHENBERICHT

Nummer der Anmeldung

EP 02 01 7469

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| A | US 6 109 776 A (HAAS JUERGEN) 29. August 2000 (2000-08-29) * Zusammenfassung * * Spalte 11, Absatz 2 * --- | 1-4 | G06F19/00 |
| A | GUHATHAKURTA D AND STORMO G D: "Identifying target sites for cooperatively binding factors" BIOINFORMATICS, 'Online! Bd. 17, Nr. 7, Juli 2001 (2001-07), Seiten 608-621, XP002250278 Gefunden im Internet: <URL:http://bioinformatics.oupjournals.org /cgi/reprint/17/7/608> 'gefunden am 2003-08-04! * Zusammenfassung * --- | 1-4 | |
| A | TSUMURA N ET AL: "Reliable classification by double hyperspheres in pattern vector space" PATTERN RECOGNITION, PERGAMON PRESS INC. ELMSFORD, N.Y, US, Bd. 28, Nr. 10, 1. Oktober 1995 (1995-10-01), Seiten 1621-1626, XP004002557 ISSN: 0031-3203 * Zusammenfassung * ----- | 1-4 | RECHERCHIERTE SACHGEBIETE (Int.Cl.7) G06F |

Der vorliegende Recherchenbericht wurde für alle Patentansprüche erstellt

| Recherchenort | Abschlußdatum der Recherche | Prüfer |
|---|---|---|
| DEN HAAG | 6. August 2003 | Filloy García, E |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

........................................................................
& : Mitglied der gleichen Patentfamilie,übereinstimmendes Dokument

EPO FORM 1503 03.82 (P04C03)

**EP 1 388 800 A1**

| Im Recherchenbericht angeführtes Patentdokument | Datum der Veröffentlichung | Mitglied(er) der Patentfamilie | Datum der Veröffentlichung |
|---|---|---|---|
| US 6109776          A | 29-08-2000 | KEINE | |

EPO FORM P0461